# FASCICULE DE BREVET EUROPEEN

(11) **EP 0 373 077 B1**
(45) Date de publication et mention de la délivrance du brevet: **06.03.1996**
(21) Numéro de dépôt: 89420450.2
(22) Date de dépôt: 17.11.1989
(51) Int. Cl.: A61B 5/107, A61C 19/04

(54) **Procédé de corrélation des saisies tridimensionnelles d'organes humains et dispositif pour sa mise en oeuvre**
Korrelationserfassungsverfahren für dreidimensionale menschliche Organe und Durchführungseinrichtung
Process of correlation to seize three-dimensional organs and means of execution

(30) Priorité: 18.11.1988 FR 8815483
(43) Date de publication de la demande: 13.06.1990
(73) Titulaire: DURET-INVENTEUR Société civile, F-11560 FLEURY D'AUDE (FR)
(72) Inventeur: Duret, François, F-38690 Le Grand Lemps (FR); Blouin, Jean-Louis, F-38200 Vienne (FR); Dechelette, Gilles, F-69110 Ste Foy Les Lyon (FR)
(74) Mandataire: Maureau, Philippe

(56) Documents cités:
- EP-A- 0 091 876
- FR-A- 2 562 236

## Description

La présente invention a pour objet un procédé de corrélation des saisies tridimensionnelles d'organes humains et un ensemble d'éléments pour sa mise en oeuvre.

Il existe différents procédés pour saisir la forme d'un organe humain interne et externe.

Le premier consiste à utiliser une pâte d'empreinte plus ou moins élastique et de restituer la pièce par coulée du plâtre afin d'obtenir la reproduction complète dudit organe.

Un deuxième procédé décrit par MOSHABAC en 1977 (US 4 182 312) puis par BECKER (US 4 411 626) consiste à micropalper la surface d'étude et éventuellement la restituer à l'aide d'une micro-fraiseuse.

Un troisième procédé consiste à faire une lecture radiographique ou tomodensitométrique puis de repositionner chaque coupe en fonction de ce qu'étaient la position des capteurs et des récepteurs d'analyse RX.

Une quatrième méthode consiste à utiliser le principe de la RMN (Résonnance Magnétique Nucléaire) ou IRM (Imagerie RM), procédés connus, largement décrits et permettant une restitution de la pièce à l'aide d'une micro-fraiseuse à commande numérique (Information dentaire Septembre 1988).

Enfin une nouvelle méthode décrite dans un ensemble de documents précédents utilise des méthodes stéreoscopiques (ROODER) ou issues du Moiré : DURET (EP-A-0 091 876) et permet de faire une lecture tridimensionnelle des objets photographiés.

Cette dernière méthode très nouvellement introduite dans la dentisterie permet de saisir une vue avec la possibilité de corréler différentes saisies pour avoir l'ensemble de la pièce faisant l'objet de l'étude.

En particulier le seul moyen utilisé pour corréler des vues suppose la fixation de l'objet analysé et de la caméra dans des positions connues du centre de calcul et d'analyse. Ainsi il est relativement courant aujourd'hui de procéder à une rotation de l'objet face à la caméra d'analyse. Or une telle technique est difficile à mettre en oeuvre dans le cas de la saisie d'une partie de la mâchoire d'un individu, dans le cas d'une application à l'art dentaire.

La présente invention a pour but de permettre la corrélation d'un ensemble de vues tridimensionnelles dentaires ou médicales d'un objet ou de corréler plusieurs objets ayant éventuellement fait l'objet d'une corrélation eux-mêmes sans connaître a priori la position de l'objet et de la caméra au niveau du calculateur.

A cet effet, le procédé qu'elle concerne comporte les étapes suivantes :
a) réaliser une empreinte des dents antagonistes en regard de la zone de taille ;
b) placer cette empreinte sur la zone de la dent taillée et sur les dents environnantes ;
c) déterminer trois points de référence sur cette zone ;
d) effectuer la corrélation des dents antagonistes à l'aide de ces trois points en prenant en considération l'empreinte de celles-ci ;
e) retirer l'élément comportant l'empreinte ;
f) effectuer la corrélation des dents de l'arcade où est taillée la dent, de manière que ces trois points soient tous visibles sur les vues obtenues par les différentes saisies et de manière à effectuer un calcul de corrélation des vues obtenues par les différentes saisies pour les ramener dans un même référentiel en tenant compte de ces trois points de référence.

Avantageusement, ce procédé consiste à placer trois points de référence sur la zone de la dent taillée et des dents environnantes.

Conformément à un mode de mise en oeuvre, chaque point de référence est supporté par un support constitué par une surface délimitée par un cadre contrasté et le procédé comporte en outre les étapes suivantes :
. évaluer la distance du point de référence par rapport à une caméra d'analyse,
. déterminer le centre du point de référence par une opération de squelettisation progressive.

Avantageusement, et afin d'éviter les inconvénients dûs à la détermination précise d'un point sur un plan en visée rasante, chaque surface associée à un point de repère est constituée par une surface de forme géométrique simple permettant d'obtenir par calcul un point caractéristique virtuel ou réel pouvant servir de base de corrélation.

Selon une possibilité, la surface associée à un point de repère est constituée par une sphère, dont le point de repère est le centre.

Cette solution est intéressante, car une sphère est une surface facile à reconnaître, et présentant la même forme et la même surface, quel que soit l'angle sous lequel elle est vue.

Avantageusement, le procédé consiste à réaliser une détermination automatique des points de référence par reconnaissance préalable de la forme des surfaces associées aux trois points, ce qui évite les risques d'erreur de corrélation manuelle.

En effet, la détermination des coordonnées de la saisie ne dépend pas des points de corrélation. Il est donc possible de connaître la position d'une sphère par rapport au centre optique de la caméra. Connaissant cette valeur, il est possible de décrire le grandissement et par suite la sphéricité et le centre de la sphère théorique.

Par ailleurs, un calcul rapide effectué sur ces points avant de calculer l'image permet d'informer immédiatement l'opérateur sur la qualité des prises de vues (bougé, codage...).

Conformément à un mode préférentiel de mise en oeuvre, les points de référence sont disposés à la périphérie de la zone dont la saisie doit être effectuée, ce qui minimise l'erreur de corrélation, puisque l'effet de bras de lever est plus réduit dans la zone centrale que dans la zone périphérique.

Dans le cas de son application à l'art dentaire, le procédé consiste à utiliser comme points de référence, des points caractéristiques de l'image, tels que sommets des cuspides ou des sillons.

Il est à noter que le repérage par trois points permet un ensemble d'autres avantages à savoir :
- l'indication manuelle d'un seul point et toujours le même permet une numérotation directe et automatique (par exemple sens des aiguilles d'une montre) des autres points. Ainsi le praticien pourra pointer le point vestibulaire dans une bouche.
- le fait d'indiquer au moins un point peut permettre au logiciel d'estimer la forme qu'il a à retrouver automatiquement et son grandissement à peu près (la zone de netteté limite les variations : profondeur de champ).

Lorsque l'on a à effectuer la corrélation d'images d'une mâchoire, une solution consiste à utiliser trois points de repère sur le maxillaire, et trois points de repère sur la mandibule, ce qui permet de réaliser des corrélations respectivement des images du maxillaire et des images de la mandibule.

Au moment du rapprochement des deux mâchoires un ou plusieurs points de référence, par exemple situés du côté intérieur de la mâchoire, sont masqués. Pour favoriser la stabilité des corrélations, un point supplémentaire sera placé dans le vestibule supérieur, et un autre sera placé dans le vestibule inférieur. Ces deux points assurent la reconstruction de deux groupes de trois points de référence en haut et en bas. Si cette méthode est rationnelle, et permet de réduire le nombre de points de corrélation tout en permettant de faire des corrélations intra-objet et inter-objet, elle demeure complexe.

Un ensemble d'éléments pour la mise en oeuvre de ce procédé comprend en combinaison :
- un élément en pâte destiné à réaliser une empreinte de la partie de la mâchoire située en regard de la partie comportant la zone de taille,
- un support pour trois points de référence, constitué par deux bras, destinés à être disposés du côté des faces linguale et vestibulaire, dans la zone taille, ce support étant destiné à être fixé de façon amovible sur la partie de la mâchoire dont l'image doit être fournie, et
- une caméra d'analyse, destinée à réaliser des saisies d'images sous différents angles.

Conformément à une forme d'exécution de ce dispositif, les surfaces auxquelles sont associés les points de référence sont fixées sur deux bras solidaires d'un arceau élastique apte à exercer une pression sur au moins une dent, ces deux bras étant disposés du côté des faces linguale et vestibulaire.

Conformément à une autre forme d'exécution, les surfaces auxquelles sont associés les points de référence sont solidaires de deux bras distincts qui, destinés à être disposés respectivement sur les faces linguale et vestibulaire, sont équipés de moyens de fixation, tels que par collage.

Conformément à une autre forme d'exécution intéressante à mettre en oeuvre pour réaliser la saisie optique d'une partie importante de la bouche, le dispositif support des points de référence comprend une barre s'étendant le long des faces linguale et vestibulaire des dents d'une mâchoire, équipée de moyens de fixation sur ladite mâchoire, par exemple par engagement de coins entre deux dents, et sur laquelle sont montées coulissantes et blocables dans la position souhaitée, des tiges supports des surfaces auxquelles sont associés les différents points de référence. Les moyens de blocage peuvent être obtenus par un serrage par vis.

De toute façon, l'invention sera bien comprise à l'aide de la description qui suit en référence au dessin schématique annexé représentantà titre d'exemples non limitatifs, plusieurs formes d'exécutions pour la mise en oeuvre de ce procédé :
Figure 1 est une vue très schématique d'un agencement utilisé pour la corrélation d'une zone d'une mâchoire ;
Figures 2 à 4 sont trois vues en perspective de trois dispositifs supports de points de référence :
Figures 5 et 6 sont deux vues de détail du dispositif de figure 5 ;
Figures 7 à 11 représentent très schématiquement un dispositif permettant de réaliser la corrélation d'images entre les deux parties de la mâchoire d'un individu.

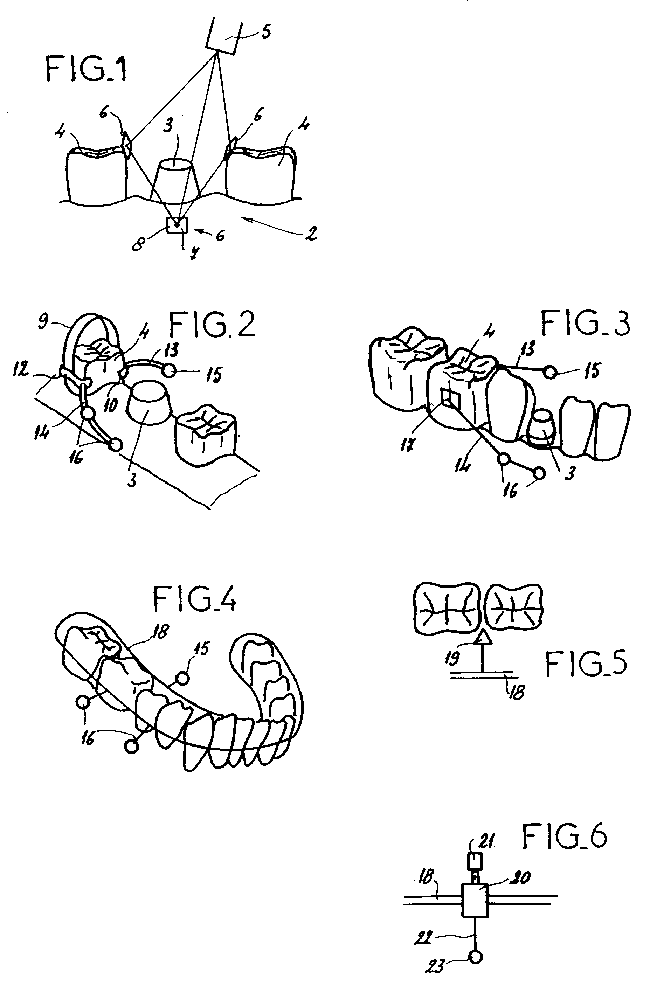

A la figure 1 est représentée très schématiquement une partie 2 d'une arcade dentaire comprenant un moignon 3 disposé entre deux dents saines 4. Pour réaliser plusieurs saisies optiques sous différents angles de cette zone, à l'aide d'une caméra d'analyse 5, il convient de placer, de préférence en périphérie de la zone comprenant le moignon 3, trois points de référence 6 dont chacun appartient à une surface 7 bordée par un cadre de couleur contrastée 8. Les trois points 6 sont, comme il a été indiqué précédemment, disposés autour du moignon, de façon à encadrer celui-ci.

La figure 2 représente un dispositif constitué par un étrier 9 réalisé en un matériau élastique, destiné à venir enserrer une dent 4. Cet étrier porte deux pattes 10, 12 prenant appui contre les faces linguale et vestibulaire de la dent et portant respectivement deux bras 13, 14. Le bras 13 est équipé d'une sphère 15 permettant la détermination d'un point de référence, tandis que le bras 14 est équipé de deux sphères 16 permettant la détermination de deux autres points de référence.

La figure 3 représente une variante d'exécution dans laquelle les bras 13 et 14 portant les sphères 15 et 16 respectivement, sont équipés chacun d'une pastille adhésive 17 destinée à être fixée sur la face d'une dent respectivement la face linguale ou vestibulaire.

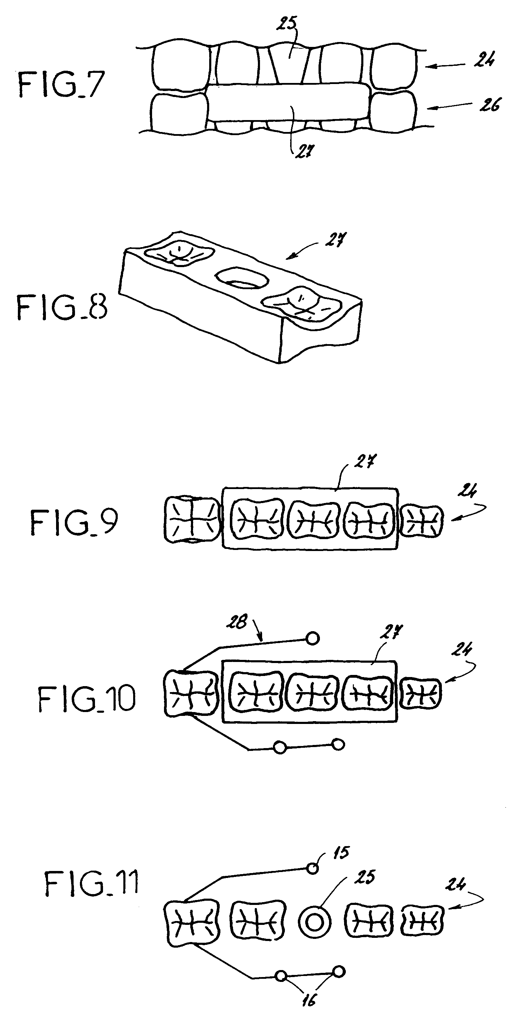

La figure 4 représente un dispositif comprenant une barre 18 refermée sur elle-même, destinée à passer le long des faces linguale et vestibulaire, et fixée par exemple vis-à-vis de la mâchoire par l'intermédiaire de coins 19 engagés entre deux dents, comme montré à la figure 6.

Comme montré à la figure 6, sur la barre 18 sont montés coulissants, avec possibilité de blocage, par une vis 21, des cavaliers 20, dont chacun porte une tige 22 dont l'extrémité libre est équipée d'une sphère 23 destinée à déterminer un point de référence.

Les figures 7 à 11 représentent schématiquement différentes phases d'un procédé permettant la corrélation de l'image des deux parties d'une mâchoire, à savoir une partie supérieure 24, comportant une dent taillée 25 destinée à recevoir une prothèse, et une partie inférieure 26. Il est procédé à la prise d'empreinte de la partie inférieure 26 de la mâchoire à l'aide d'une pâte 27, par la technique dite du "mordu". L'élément en pâte 27 est ensuite positionné sur la partie 24 de la mâchoire, comportant le moignon 25. Sur cette partie de la mâchoire est placé un système 28 comportant des points de référence, constitué par exemple par le système représenté à la figure 3. Il est alors procédé, comme montré à la figure 11, à une corrélation en tenant compte de la partie en creux de l'empreinte 27 qui est le négatif de la partie 26 de la mâchoire antagoniste à la zone de la prothèse.

L'élément en pâte 27 est ensuite retiré, et il est procédé comme montré à la figure 12 à des saisies optiques de la zone de la prothèse elle-même. Il est intéressant de noter que la corrélation au niveau de chaque partie de la mâchoire et entre les deux parties de la mâchoire, est réalisée à l'aide des mêmes points de référence.

Comme il ressort de ce qui précède, l'invention apporte une grande amélioration à la technique existante, en fournissant un procédé de conception simple permettant la corrélation de saisies tridimensionnelles d'organes humains, d'une grande fiabilité et supprimant toutes difficultés pour le praticien.

Comme il va de soi, l'invention ne se limite pas aux seuls modes de mise en oeuvre de ce procédé, ni aux seules formes d'exécution de ce dispositif, décrits ci-dessus à titre d'exemples ; elle en embrasse, au contraire, toutes les variantes de réalisation, c'est ainsi notamment que la forme des surfaces à partir desquelles sont définis les points de référence pourrait être différente d'un plan ou d'une sphère, sans que l'on sorte pour autant du cadre de l'invention.

## Revendications

1. Procédé de corrélation de saisies tridimensionnelles d'organes humains, dans le cas de la corrélation des deux parties d'une mâchoire, comportant les étapes suivantes, dans le cas de la réalisation d'une prothèse :
a) réaliser une empreinte (27) des dents antagonistes (26) en regard de la zone de taille (24) ;
b) placer cette empreinte (27) sur la zone de la dent taillée et sur les dents environnantes ;
c) déterminer trois points de référence (6) sur cette zone ;
d) effectuer la corrélation des dents antagonistes à l'aide de ces trois points (15, 16) en prenant en considération l'empreinte de celles-ci ;
e) retirer l'élément comportant l'empreinte (27) ;
f) effectuer la corrélation des dents de l'arcade où est taillée la dent, de manière que ces trois points (6) soient tous visibles sur les vues obtenues par les différentes saisies et de manière à effectuer un calcul de corrélation des vues obtenues par les différentes saisies pour les ramener dans un même référentiel en tenant compte de ces trois points de référence.

2. Procédé selon la revendication 1, caractérisé en ce qu'il consiste à placer trois points de référence (6) sur la zone de la dent taillée et des dents environnantes.

3. Procédé selon l'une des revendications 1 et 2, caractérisé en ce que chaque point de référence (6) est supporté par un support constitué par une surface délimitée par un cadre contrasté et en ce qu'il comporte en outre les étapes suivantes :
. évaluer la distance du point de référence (6) par rapport à une caméra (5) d'analyse,
. déterminer le centre du point de référence (6) par une opération de squelettisation progressive.

4. Procédé selon la revendication 3, caractérisé en ce que la surface (27) associée à un point de référence (6) est constituée par une sphère (15, 16) dont le point de référence (6) est le centre.

5. Procédé selon l'une quelconque des revendications 1 à 4, caractérisé en ce que les points de référence (6) sont disposés à la périphérie de la zone dont la saisie doit être effectuée.

6. Ensemble d'éléments pour la mise en oeuvre d'un procédé selon l'une des revendications 1 à 5, comprenant en combinaison :
- un élément en pâte destiné à réaliser une empreinte (27) de la partie de la mâchoire située en regard de la partie comportant la zone de taille (24),
- un support pour trois points de référence (6), constitué par deux bras (13, 14), destinés à être disposés du côté des faces linguale et vestibulaire, dans la zone taille (24), ce support étant destiné à être fixé de façon amovible sur la partie de la mâchoire dont l'image doit être fournie, et
- une caméra d'analyse (5), destinée à réaliser des saisies d'images sous différents angles.

7. Ensemble selon la revendication 6, caractérisé en ce que les bras (13, 14) sont solidaires d'un arceau élastique (9) apte à exercer une pression sur au moins une dent.

8. Ensemble selon la revendication 6, caractérisé en ce que les bras sont distincts et sont équipés de moyens de fixation (17), tels que par collage.

9. Ensemble selon la revendication 6, caractérisé en ce que les bras sont constitués par une barre (18) s'étendant le long des faces linguale et vestibulaire des dents d'une mâchoire, équipée de moyens de fixation sur ladite mâchoire, par exemple par engagement de coins entre deux dents, et sur laquelle sont montées coulissantes et blocables dans la position souhaitée, des tiges supports des surfaces auxquelles sont associés les différents points de référence (6).

## Claims

1. A procedure for correlating three-dimensional recordings of human organs in the case of the correlation of the two parts of a jaw, comprising the following stages in the case of the production of a prosthesis:
a) producing an impression (27) of the opposing teeth (26) opposite the cutting area (24);
b) placing this impression (27) on the area of the cut tooth and on the surrounding teeth;
c) determining three reference points (6) on this area;
d) correlating the opposing teeth with the aid of these three points (6), by taking into consideration the impression of the latter;
e) removing the element comprising the impression (27);
f) correlating the teeth of the arch where the tooth is cut in such a way that the three points (6) are all visible in the views obtained by the different recordings and so as to perform a correlation calculation of the views obtained by the different recordings in order to bring them within the same reference frame by taking into account these three reference points.

2. A procedure according to claim 1, characterised in that it consists of placing three reference points (6) on the area of the cut tooth and of the surrounding teeth.

3. A procedure according to one of claims 1 and 2, characterised in that each reference point (6) is supported by a support formed by a surface delimited by a contrasting frame, and that the procedure additionally comprises the following stages:
. evaluating the distance of the reference point (6) with respect to an analysis camera (5),
. determining the centre of the reference point (6) by a progressive skeletalisation operation.

4. A procedure according to claim 3, characterised in that the surface (27) associated with a reference point (6) is formed by a sphere (15, 16), the centre of which is the reference point (6).

5. A procedure according to any one of claims 1 to 4, characterised in that the reference points (6) are disposed at the periphery of the area of which the recording is to be taken.

6. An assembly of elements for carrying out a procedure according to any one of claims 1 to 5, comprising, in combination:
- an element made of paste intended to produce an impression (27) of the part of the jaw situated opposite the part comprising the cutting zone (24),
- a support for three reference points (6), formed from two arms (13, 14) intended to be disposed at the side of the lingual and vestibular surfaces, within the cutting area (24), the said support being intended to be fixed in a relocatable manner on the part of the jaw of which the image is to be provided, and
- an analysis camera (5) intended to make recordings of images at different angles.

7. An assembly according to claim 6, characterised in that the arms (13, 14) are an integral part of an elastic hoop (9) capable of exerting a pressure on at least one tooth.

8. An assembly according to claim 6, characterised in that the arms are separate and are equipped with means of fixation (17), such as by adhesive bonding.

9. An assembly according to claim 6, characterised in that the arms are formed from a bar (18) extending along the lingual and vestibular surfaces of the teeth of a jaw and equipped with means of fixation to the said jaw, for example by the engagement of wedges between two teeth, on which bar support rods for the surfaces with which the different reference points (6) are associated are mounted so that they can slide and can be locked in the desired position.

## Patentansprüche

1. Verfahren zur dreidimensionalen Korrelationserfassung von Teilen eines Menschen im Falle der Korrelation zweier Teile eines Kiefers, umfassend die folgenden Schritte im Falle der Fertigung einer Prothese:
a) Bilden eines Abdrucks (27) der dem abgeschliffenen Bereich (24) gegenüberliegenden Gegenzähne (26);
b) Anordnen dieses Abdrucks (27) auf dem Bereich des abgeschliffenen Zahns und auf den benachbarten Zähnen;
c) Bestimmen von drei Bezugspunkten (6) auf diesem Bereich;
d) Durchführen der Korrelation der Gegenzähne mit Hilfe dieser drei Punkte (15, 16) unter Berücksichtigung des Abdrucks derselben;
e) Abnehmen des den Abdruck (27) umfassenden Elements;
f) Durchführen der Korrelation der Zähne des Bogens, in dem sich der abgeschliffene Zahn befindet, so daß diese drei Punkte (6) auf den durch verschiedene Erfassungen erhaltenen Ansichten alle sichtbar sind und um eine Korrelationsrechnung der durch die verschiedenen Erfassungen erhaltenen Ansichten durchzuführen, um sie unter Berücksichtigung dieser drei Bezugspunkte in ein und dasselbe Bezugssystem zurückzuführen.

2. Verfahren nach Anspruch 1,
**dadurch gekennzeichnet**, daß es darin besteht, drei Bezugspunkte (6) auf dem Bereich des abgeschliffenen Zahns und der benachbarten Zähnen anzuordnen.

3. Verfahren nach einem der Ansprüche 1 und 2,
**dadurch gekennzeichnet**, daß jeder Bezugspunkt (6) von einem Träger gehalten wird, der von einer von einem kontrastierten Rahmen begrenzten Fläche gebildet ist, und daß es darüber hinaus die folgenden Schritte umfaßt:
. Auswerten des Abstands des Bezugspunktes (6) bezüglich einer Analysekamera (5),
. Bestimmen des Zentrums des Bezugspunktes (6) mittels einer fortschreitenden Zerlegungsoperation.

4. Verfahren nach Anspruch 3,
**dadurch gekennzeichnet**, daß die einem Bezugspunkt (6) zugeordnete Fläche (27) von einer Kugel (15, 16) gebildet wird, deren Zentrum der Bezugspunkt (6) ist.

5. Verfahren nach einem der Ansprüche 1 bis 4,
**dadurch gekennzeichnet**, daß die Bezugspunkte (6) auf dem Umfang des Bereichs angeordnet sind, dessen Erfassung durchgeführt werden soll.

6. Anordnung von Elementen zur Durchführung eines Verfahrens nach einem der Ansprüche 1 bis 5, umfassend in Kombination:
- ein teigiges Element, das zur Bildung eines Abdrucks (27) des Abschnitts des Kiefers bestimmt ist, der dem den abgeschliffenen Bereich (24) umfassenden Abschnitt gegenüberliegt,
- eine Halterung für drei Bezugspunkte (6), die von zwei Schenkeln (13, 14) gebildet ist, welche zur Anordnung in dem abgeschliffenen Bereich (24) auf den lingualen und vestibulären Seiten bestimmt sind, wobei diese Halterung zur abnehmbaren Befestigung auf dem Abschnitt des Kiefers bestimmt ist, dessen Bild geliefert werden soll, und
- eine Analysekamera (5), die zur Durchführung der Bilderfassungen unter verschiedenen Winkeln bestimmt ist.

7. Anordnung nach Anspruch 6,
**dadurch gekennzeichnet**, daß die Schenkel (13, 14) mit einem elastischen kleinen Bogen (9) verbunden sind, der auf wenigstens einen Zahn einen Druck ausüben kann.

8. Anordnung nach Anspruch 6,
**dadurch gekennzeichnet**, daß die Schenkel voneinander getrennt sind und mit Mitteln (17) zur Befestigung, bspw. mittels Kleben, ausgestattet sind,

9. Anordnung nach Anspruch 6,
**dadurch gekennzeichnet**, daß die Schenkel von einer Stange (18) gebildet sind, welche sich längs der lingualen und vestibulären Seiten der Zähne eines Kiefers erstreckt, welche mit Mitteln zur Befestigung an dem Kiefer, bspw. durch Eingriff von Spitzen zwischen zwei Zähne, ausgestattet ist und auf welcher verschiebbar und in der gewünschten Stellung blockierbar Schafte zur Halterung der Flächen angebracht sind, welchen die verschiedenen Bezugspunkte (6) zugeordnet sind.
